# EUROPÄISCHE PATENTSCHRIFT

(11) **EP 2 285 791 B1**
(45) Veröffentlichungstag und Bekanntmachung des Hinweises auf die Patenterteilung: **07.03.2012**
(21) Anmeldenummer: 08873464.5
(22) Anmeldetag: 16.12.2008
(51) Int. Cl.: C07D 301/12

(54) **VERFAHREN ZUR HERSTELLUNG VON EPICHLORHYDRIN**
METHOD FOR THE PRODUCTION OF EPICHLOROHYDRIN
PROCÉDÉ DE FABRICATION D'ÉPICHLORHYDRINE

(30) Priorität: 17.03.2008 EP 08102653
(43) Veröffentlichungstag der Anmeldung: 23.02.2011
(73) Patentinhaber: Momentive Specialty Chemicals GmbH, 58642 Iserlohn-Letmathe (DE)
(72) Erfinder: HOFEN, Willi, 63517 Rodenbach (DE); BRASSE, Claudia, 63452 Hanau (DE); FRANKE, Robert, 45772 Marl (DE); KATZER, Robert, 60596 Frankfurt Am Main (DE)
(86) Internationale Anmeldenummer: PCT/EP2008/067584
(87) Internationale Veröffentlichungsnummer: WO 2009/115152

(56) Entgegenhaltungen:
- WO-A-2004/048353
- US-A1- 2003 187 285
- WANG LINGLING, LIU YUEMING, ZHANG HAIJIAO, JIANG YONGWEN, WU PENG, HE MINGYUAN: "Highly Efficient Synthesis of Epichlorohydrin by Epoxidation of Allyl Chloride over Titanosilicate Ti-NWW" CHINESE JOURNAL OF CATALYSIS, Bd. 27, Nr. 8, 1. August 2006 (2006-08-01), Seiten 656-658, XP002484781

## Beschreibung

Die Erfindung betrifft ein Verfahren zur Herstellung von Epichlorhydrin durch Umsetzung von Allylchlorid mit Wasserstoffperoxid.

Epichlorhydrin (Chlormethyloxiran) ist ein wichtiges chemisches Zwischenprodukt, das z. B. zur Herstellung von Harzen verwendet wird.

Ein zur Herstellung von Epichlorhydrin geeignetes Verfahren ist die aus EP-A 0 100 119 bekannte Umsetzung von Allylchlorid mit Wasserstoffperoxid in Gegenwart eines titanhaltigen Zeolithkatalysators. Um bei dieser Reaktion eine hohe Selektivität für Epichlorhydrin zu erhalten, muss Allylchlorid in stöchiometrischem Überschuss zu Wasserstoffperoxid eingesetzt werden, wie beispielsweise aus WO 2004/043941 bekannt ist. Nicht umgesetztes Allylchlorid kann durch Destillation abgetrennt und in die Epoxidierungsreaktion zurückgeführt werden, wie z. B. aus WO 02/00634 oder WO 02/14298 bekannt ist.

Weitere Verfahren zur Epoxidierung von Allylchlorid sind in der WO 2004/048353, der US 2003/187285 und in Wang Lingling, Liu Yueming, Zhang Haijiao, Jiang Yongwen, Wu Peng, He Mingyuan:"Highly Efficient Synthesis of Epichorhydrine by Epoxidation of Allyl Chloride over Titanosilicate Ti-NWw", Chinese Journal of Catalysis Bd. 27, Nr. 8, 1. August 2006 (2006-08-01), Seiten 656-658, XP002484781 beschrieben.

Technisches Allylchlorid ist in der Regel mit 1-Chlorpropan und/oder 2-Chlorpropan verunreinigt. Beide Verunreinigungen haben ähnlich Siedepunkte wie Allylchlorid:
Allylchlorid: 45°C
1-Chlorpropan: 47°C
2-Chlorpropan: 36°C

Die beiden Chlorpropane lassen sich von Allylchlorid deshalb nur mit großem Aufwand destillativ abtrennen. Wenn für die Umsetzung von Allylchlorid mit Wasserstoffperoxid ein technisches, mit Chlorpropanen verunreinigtes Allylchlorid eingesetzt wird und nicht umgesetztes Allylchlorid durch Destillation abgetrennt und in die Epoxidierungsreaktion zurückgeführt wird, kommt es demnach zu einer Anreicherung von Chlorpropanen im Verfahren.

Aus WO 02/00634 und WO 02/14298 ist bekannt, dass sich eine Anreicherung von Verunreinigungen im zurückgeführten, nicht umgesetzten Olefin auf unerwünscht hohe Konzentrationen durch die Ausschleusung eines Teils des zurückgeführten Olefins aus dem Verfahren verhindern lässt. Der ausgeschleuste Teilstrom enthält allerdings einen hohen Anteil an Olefin, der durch die Ausschleusung verloren geht.

Es besteht deshalb ein Bedarf nach einem Verfahren zur Herstellung von Epichlorhydrin durch Umsetzung eines Chlorpropane enthaltenden Allylchlorids mit einer hohen Selektivität für Epichlorhydrin und einer gegenüber den bekannten Verfahren verbesserten Umsetzung des eingesetzten Allylchlorids.

Diese Aufgabe wird gelöst durch ein erfindungsgemäßes Verfahren, bei dem in einer ersten Reaktionsstufe ein Chlorpropane enthaltendes Allylchlorid im Überschuss mit Wasserstoffperoxid umgesetzt wird. Das nicht umgesetzte Allylchlorid wird abgetrennt und in die Reaktion zurückgeführt wird, wobei ein Teil des abgetrennten Allylchlorids einer zweiten Reaktionsstufe zugeführt und mit Wasserstoffperoxid umgesetzt wird, wobei die Wasserstoffperoxidmenge in der zweiten Reaktionsstufe so gewählt ist, dass das Allylchlorid weitgehend und vorzugsweise praktisch vollständig umgesetzt wird. Aus der Reaktionsmischung der zweiten Reaktionsstufe lassen sich die Chlorpropane dann ohne Verluste an Allylchlorid durch Destillation abtrennen. Da in dem erfindungsgemäßen Verfahren der größte Teil des Allylchlorids bei einem Überschuss an Allylchlorid umgesetzt wird und nur ein kleiner Teil des Allylchlorids bei einem geringen Überschuss oder einem Unterschuss an Allylchlorid reagiert, bleibt die durch einen Überschuss an Allylchlorid bewirkte hohe Selektivität für Epichlorhydrin erhalten.

Gegenstand der Erfindung ist deshalb ein Verfahren zur Herstellung von Epichlorohydrin, bei dem
a) in einer ersten Reaktionsstufe Allylchlorid und Wasserstoffperoxid in Gegenwart eines titanhaltigen Zeolithkatalysators in einem molaren Verhältnis von Allylchlorid zu Wasserstoffperoxid von mindestens 1,5:1 umgesetzt werden und wobei das eingesetzte Allylchlorid 1-Chlorpropan und/oder 2-Chlorpropan enthält,
b) die in der ersten Reaktionsstufe gebildete Reaktionsmischung in einer Destillation aufgetrennt wird in eine Mischung (A), die nicht umgesetztes Allylchlorid, sowie 1-Chlorpropan und/oder 2-Chlorpropan enthält und eine Mischung (B), die Epichlorhydrin enthält,
c) die Mischung (A) aufgeteilt wird in eine Mischung (A1), die in die erste Reaktionsstufe zurückgeführt wird und eine Mischung (A2),
d) die Mischung (A2) in einer zweiten Reaktionsstufe mit Wasserstoffperoxid in Gegenwart eines titanhaltigen Zeolithkatalysators in einem molaren Verhältnis von Allylchlorid zu Wasserstoffperoxid im Bereich von 0,5:1 bis 1,25:1 umgesetzt wird,
e) die in der zweiten Reaktionsstufe gebildete Reaktionsmischung in einer Destillation aufgetrennt wird in eine Mischung (C), die 1-Chlorpropan und/oder 2-Chlorpropan enthält und eine Mischung (D), die Epichlorhydrin enthält und
f) die Mischung (C) aus dem Verfahren entnommen wird.

Bei dem erfindungsgemäßen Verfahren werden Allylchlorid und Wasserstoffperoxid in Gegenwart eines titanhaltigen Zeolithkatalysators zu Epichlorhydrin umgesetzt. Das eingesetzte Allylchlorid enthält dabei 1-Chlorpropan und/oder 2-Chlorpropan. Für das erfindungsgemäße Verfahren können deshalb technische Qualitäten von Allylchlorid verwendet werden, die 1-Chlorpropan und/oder 2-Chlorpropan als Nebenprodukte der technischen Herstellung von Allylchlorid enthalten. Der Gehalt an 1-Chlorpropan und 2-Chlorpropan im eingesetzten Allylchlorid liegt vorzugsweise im Bereich von 0,01 bis 2 Gew.-%, besonders bevorzugt im Bereich von 0,05 bis 0,8 Gew.-%.

Wasserstoffperoxid kann als wässrige Lösung eingesetzt werden, die vorzugsweise einen Gehalt an Wasserstoffperoxid im Bereich von 1 bis 90 Gew.-%, besonders bevorzugt von 10 bis 80 Gew.-% und insbesondere von 30 bis 70 Gew.-%, aufweist. Wasserstoffperoxid kann als handelsübliche, stabilisierte Lösung eingesetzt werden. Ebenso geeignet ist nach dem Anthrachirtonverfahren hergestelltes, nicht stabilisiertes Wasserstoffperoxid, das ohne weitere Reinigung eingesetzt werden kann. Vorzugsweise wird ein aus WO 2004/028962 bekanntes Wasserstoffperoxid verwendet, dass weniger als 50 ppm Alkalimetalle und Erdalkalimetalle, weniger als 50 ppm Basen mit einem pK_{B} von weniger als 4,5 und mindestens 100 ppm Anionen, jeweils bezogen auf das Gewicht an Wasserstoffperoxid, enthält.

In einer weiteren bevorzugten Ausführungsform wird eine Lösung von Wasserstoffperoxid in Methanol verwendet, die vorzugsweise durch Umsetzung von Wasserstoff und Sauerstoff an einem Palladiumkatalysator in Methanol hergestellt wurde. Besonders bevorzugt wird eine Wasserstoffperoxidlösung in Methanol gemäß Anspruch 9 von WO 2006/108784 verwendet, die 2 bis 15 Gew.-% Wasserstoffperoxid, 0,5 bis 20 Gew.-% Wasser, 60 bis 95 Gew.-% Methanol, 10⁻⁶ bis 10⁻² mol/l Bromid, sowie 10⁻⁶ bis 0,1 mol/l Dimethylsulfat und/oder Monomethylsulfat enthält.

Als titanhaltiger Zeolithkatalysator können alle aus dem Stand der Technik bekannten titanhaltigen Zeolithe verwendet werden, die eine katalytische Aktivität für die Umsetzung von Olefinen mit Wassesrtoffperoxid aufweise. Vorzugweise wird als titanhaltiger Zeolithkatalysator ein Titansilicalit mit MFI- oder MEL-Kristallstruktur eingesetzt. Besonders bevorzugt werden Titansilicalite der Zusammensetzung (TiO₂)ₓ(SiO₂)₁₋ₓ verwendet, wobei x im Bereich von 0,001 bis 0,05 liegt. Am meisten bevorzugt sind Titansilicalite, die nach dem Verfahren gemäß WO 01/64581 oder dem Verfahren gemäß WO 01/64582 hergestellt wurden.

Der titanhaltige Zeolithkatalysator kann im erfindungsgemäßen Verfahren in Form eines Suspensionskatalysators verwendet werden. In diesem Fall wird die Umsetzung bevorzugt so durchgeführt, dass der in der Reaktionsmischung suspendierte Katalysator in der ersten Reaktionsstufe zurückgehalten wird, beispielsweise durch Filtration oder durch Sedimentation, so dass die Reaktionsmischung, die in Schritt b) in einer Destillation aufgetrennt wird, keinen Katalysator enthält.

Vorzugsweise wird der titanhaltige Zeolithkatalysator im erfindungsgemäßen Verfahren jedoch in Form eines Festbettkatalysators verwendet. Besonders geeignet sind durch Extrusion verformte Festbettkatalysator.en in Form von Extrudaten mit einem Durchmesser von 1 bis 5 mm, die vorzugsweise ein Bindemittel in einer Menge von 1 bis 99 Gew.-%, besonders bevorzugt 1 bis 40 Gew.-%, bezogen auf den titanhaltigen Zeolith enthalten. Geeignet sind dabei alle Bindemittel, die unter den Reaktionsbedingungen weder mit dem eingesetzten Wasserstoffperoxid noch mit dem gebildeten Epichlorhydrin reagieren. Besonders geeignete Bindemittel sind Kieselsäuren. Besonders bevorzugt sind Festbettkatalysatoren, bei denen zur Extrusion eine pyrogene Kieselsäure, ein kolloidales Kieselsol oder ein Tetraalkylorthosilikat oder eine Kombination von zwei dieser Komponenten als Vorläufer für das Bindemittel eingesetzt wurden. Ebenfalls besonders bevorzugt sind Festbettkatalysatoren, die nach dem aus WO 01/72419 bekannten Verfahren durch Verformen einer Formmasse hergestellt wurden, die einen Plateauwert der Curdkurve im Bereich von 20 bis 90 mm aufweist.

Bei dem erfindungsgemäßen Verfahren werden in Schritt a) in der ersten Reaktionsstufe Allylchlorid und Wasserstoffperoxid in einem molaren Verhältnis von Allylchlorid zu Wasserstoffperoxid von mindestens 1,5:1 umgesetzt. Das molare Verhältnis von Allylchlorid zu Wasserstoffperoxid kann dabei bis zu 100:1 betragen. Vorzugsweise liegt das molare Verhältnis im Bereich von 1,5:1 bis 5:1. Besonders bevorzugt beträgt das molare Verhältnis von Allylchlorid zu Wasserstoffperoxid 2:1 bis 4:1. Bei einem kleineren molaren Verhältnis nimmt die Selektivität für Epichlorhydrin in der ersten Reaktionsstufe ab. Höhere molare Verhältnisse haben den Nachteil, dass große Mengen an nicht umgesetztem Allylchlorid mit entsprechendem Energieaufwand abgetrennt und zurückgeführt werden müssen.

In Schritt d) des erfindungsgemäßen Verfahrens werden in der zweiten Reaktionsstufe Allylchlorid und Wasserstoffperoxid in einem molaren Verhältnis von Allylchlorid zu Wasserstoffperoxid im Bereich von 0,5:1 bis 1,25:1 umgesetzt. Vorzugsweise beträgt das molare Verhältnis von Allylchlorid zu Wasserstoffperoxid 0,8:1 bis 1,15:1. Die Verwendung eines molaren Verhältnisses in diesen Bereichen ermöglichter, in der zweiten Reaktionsstufe Allylchlorid ganz oder zum größten Teil umzusetzen, so dass die in Schritt e) erhaltene und in Schritt f) aus dem Verfahren entnommene Mischung (C) nur einen geringen Anteil des eingesetzte Allylchlorids enthält.

Die Umsetzung von Allylchlorid und Wasserstoffperoxid erfolgt in den Schritten a) und d) vorzugsweise in Gegenwart eines Lösungsmittels. Besonders geeignet sind Lösungsmittel, die unter den Reaktionsbedingungen Allylchlorid und Wasserstoffperoxid lösen und nicht oder nur in einem geringen Maß mit Wasserstoffperoxid oder Epichlorhydrin reagieren. Als Lösungsmittel eignen sich beispielsweise Alkohole, wie Methanol, Ethanol oder tert-Butanol; Glykole, wie beispielsweise Ethylenglykol, 1,2-Propandiol oder 1,3-Propandiol; acyclische Ether, wie beispielsweise Tetrahydrofuran oder Dioxan; Glykolether, wie beispielsweise Ethylenglykolmonomethylether, Ethylenglykolmonoethylether, Ethylenglykolrrionobutylether oder Propylenglykolmonomethylether; sowie Ketone, wie beispielsweise Aceton oder 2-Butanon. Bevorzugte Lösungsmittel sind aliphatische Alkohole mit 1 bis 4 Kohlenstoffatomen. Besonders bevorzugt wird Methanol als Lösungsmittel verwendet. Der Anteil an Lösungsmittel an der Reaktionsmischung beträgt in der ersten Reaktionsstufe vorzugsweise 10 bis 95 Gew.-%, besonders bevorzugt 30 bis 80 Gew.-% und in der zweiten Reaktionsstufe vorzugsweise 10 bis 95 Gew.-%, besonders bevorzugt 30 bis 80 Gew.-%.

Die Umsetzung von Allylchlorid und Wasserstoffperoxid erfolgt in der ersten Reaktionsstufe vorzugsweise bei einer Temperatur im Bereich von 0°C bis 100°C, besonders bevorzugt 30°C bis 65°C und in der zweiten Reaktionsstufe vorzugsweise bei einer Temperatur im Bereich von 0°C bis 100°C, besonders bevorzugt 30°C bis 65°C. Der Druck in den Reaktionsstufen kann in weiten Grenzen frei gewählt werden und wird vorzugsweise so hoch gewählt, dass der Siedepunkt von Allylchlorid bei dem verwendeten Druck gleich oder höher ist als die verwendete Reaktionstemperatur.

Die Reaktionsbedingungen werden in der ersten Reaktionsstufe vorzugsweise so gewählt, dass ein Umsatz von Wasserstoffperoxid im Bereich von 50% bis 100%, vorzugsweise von 80% bis 99,8%, erreicht wird. In der zweiten Reaktionsstufe werden die Reaktionsbedingungen vorzugsweise so gewählt, dass von den Komponenten Allylchlorid und Wasserstoffperoxid die im molaren Unterschuss eingesetzte Komponente zu 70% bis 100%, vorzugsweise zu 90% bis 99%, umgesetzt wird.

Zur Umsetzung von Allylchlorid und Wasserstoffperoxid in den Schritten a) und d) können alle für die Durchführung von Flüssigphasenreaktionen geeigneten Reaktoren verwendet werden. Die Umsetzung kann dabei sowohl satzweise als auch kontinuierlich erfolgen, wobei eine kontinuierliche Umsetzung bevorzugt ist.

Vorzugsweise erfolgt die Umsetzung in den Schritten a) und d) kontinuierlich in einem Festbettreaktor, wobei eine Mischung, die Wasserstoffperoxid, gegebenenfalls Lösungsmittel sowie Allylchlorid bzw. die Mischung (A2) enthält, über ein Festbett des titanhaltigen Zeolithkatalysators geleitet wird. Als Festbettreaktor wird vorzugsweise ein von außen gekühlter Rohrreaktor, insbesondere ein Rohrbündelreaktor verwendet. Der Festbettreaktor kann sowohl in Aufwärtsströmung als auch in Abwärtsströmung betrieben werden, wobei ein Betrieb mit Abwärtsströmung im Rieselbettzustand bevorzugt wird.

Sowohl in Schritt a) als auch in Schritt d) kann die Umsetzung in zwei oder mehr in Reihe geschalteten Reaktoren durchgeführt werden. Vorzugsweise werden in Schritt a) zwei in Reihe geschaltete Reaktoren verwendet. Sowohl in Schritt a) als auch in Schritt d) können zwei oder mehr parallel angeordnete Reaktoren verwendet werden, so dass ein Reaktor für eine Regenerierung des Katalysators außer Betrieb genommen werden kann und die Umsetzung in einem parallel geschalteten Reaktor fortgesetzt werden kann.

Die in der ersten Reaktionsstufe gebildete Reaktionsmischung wird in Schritt b) des erfindungsgemäßen Verfahrens in einer Destillation aufgetrennt wird in eine Mischung (A), die nicht umgesetztes Allylchlorid, sowie 1-Chlorpropane und/oder 2-Chlorpropan enthält und eine Mischung (B), die Epichlorhydrin enthält. Die Destillation wird vorzugsweise als kontinuierliche Rektifikation durchgeführt, wobei einer Rektifikationskolonne in einem mittleren Abschnitt die in der ersten Reaktionsstufe gebildete Reaktionsmischung zugeführt wird, am Kopf der Kolonne die Mischung (A) entnommen wird und aus dem Sumpf der Kolonne die Mischung (B) entnommen wird. Vorzugsweise wird eine Rektifikationskolonne mit 10 bis 50 theoretischen Trennstufen verwendet. Die Rektifikation erfolgt vorzugsweise bei einem Druck am Kolonnenkopf im Bereich von 0,2 bis 3 bar und vorzugsweise bei einem Rücklaufverhältnis von 0,5 bis 5.

Die Destillation wird vorzugsweise so betrieben, dass die resultierende Mischung (A) mehr als 95% des in der zugeführten Reaktionsmischung enthaltenen Allylchlorids enthält und die resultierende Mischung (B) mehr als 95% des in der zugeführten Reaktionsmischung enthaltenen Epichlorhydrins enthält.

In Schritt c) des erfindungsgemäßen Verfahrens wird die Mischung (A) aufgeteilt in eine Mischung (A1), die in die erste Reaktionsstufe zurückgeführt wird und eine Mischung (A2), die der zweiten Reaktionsstufe zugeführt wird.

Vorzugsweise wird die Mischung (A) so aufgeteilt, dass 50% bis 98%, besonders bevorzugt 70% bis 95% des in der Mischung (A) enthaltenen Allylchlorids mit der Mischung (A1) in die erste Reaktionsstufe zurückgeführt wird.

In einer bevorzugten Ausführungsform wird die Mischung (A) durch eine Destillation aufgetrennt, so dass die in Mischung (A) enthaltenen Chlorpropane in der Mischung (A2) angereichert werden.

In Schritt e) des erfindungsgemäßen Verfahrens wird die in der zweiten Reaktionsstufe gebildete Reaktionsmischung in einer Destillation aufgetrennt in eine Mischung (C), die 1-Chlorpropan und/oder 2-Chlorpropan enthält und eine Mischung (D), die Epichlorhydrin enthält. Die Destillation wird vorzugsweise als kontinuierliche Rektifikation durchgeführt, wobei einer Rektifikationskolonne in einem mittleren Abschnitt die in der zweiten Reaktionsstufe gebildete Reaktionsmischung zugeführt wird, am Kopf der Kolonne die Mischung (C) entnommen wird und aus dem Sumpf der Kolonne die Mischung (D) entnommen wird. Vorzugsweise wird eine Rektifikationskolonne mit 10 bis 50 theoretischen Trennstufen verwendet. Die Rektifikation erfolgt vorzugsweise bei einem Druck am Kolonnenkopf im Bereich von 0,5 bis 3 bar und vorzugsweise bei einem Rücklaufverhältnis von 0,5 bis 5.

Die Destillation wird vorzugsweise so betrieben, dass die resultierende Mischung (C) mehr als 90% der in der zugeführten Reaktionsmischung enthaltenen Chlorpropane enthält und die resultierende Mischung (D) mehr als 95% des in der zugeführten Reaktionsmischung enthaltenen Epichlorhydrins enthält.

In einer bevorzugten Ausführungsform wird die in Schritt e) des erfindungsgemäßen Verfahrens erhaltene Mischung (D) in die erste Reaktionsstufe zurückgeführt. Diese Ausführungsform ist besonders vorteilhaft, wenn in Schritt d) Wasserstoffperoxid in molarem Überschuss eingesetzt wird und die Mischung (D) noch nicht umgesetztes Wasserstoffperoxid enthält. Durch die Rückführung in die erste Reaktionsstufe kann dieses nicht umgesetzte Wasserstoffperoxid noch zur Epoxidierung von weiterem Allylchlorid genutzt werden. In einer besonders bevorzugten Ausführungsform wird die erste Reaktionsstufe in zwei in Reihe geschaltete Reaktoren durchgeführt und die mischung (D) in den zweiten Reaktor zurückgeführt.

In einer weiteren Ausführungsform erfel-gen die Schritte d) und e) des erfindungsgemäßen Verfahrens gleichzeitig in Form einer Reaktivdestillation. Bei dieser Ausführungsform wird der titanhaltige Zeolithkatalysator der zweiten Reaktionsstufe in einem Reakti-onsabschnitt einer Rektifikationskolonne angeordnet, die Mischung (A2) der Kolonne an einem Punkt unterhalb des Reaktionsabschnitts zugeführt und Wasserstoffperoxid an einem Punkt oberhalb des Reaktionsabschnitts zugeführt. Am Kopf der Kolonne wird die Mischung (C) entnommen und aus dem Sumpf der Kolonne wird die Mischung (D) entnommen.

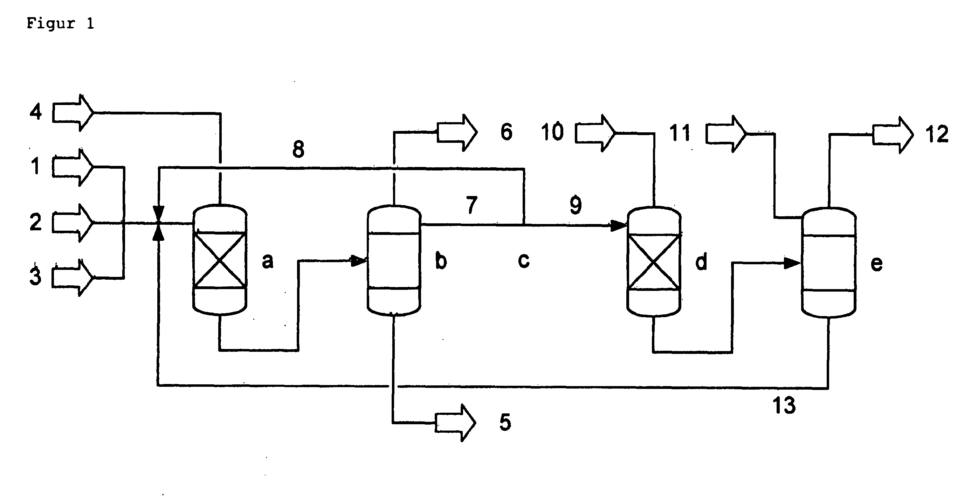

Figur 1 zeigt schematisch eine bevorzugte Ausführungsform des beanspruchten Verfahrens, bei der die Umsetzungen in den Schritten a) und d) in Festbettreaktoren erfolgen und die Destillationen in den Schritten b) und e) in Destillationskolonnen durchgeführt werden. Nicht gezeigt sind die zur Durchführung des Verfahrens erforderlichen Hilfsaggregate, wie Pumpen, Wärmetauscher, Verdampfer und Kondensatoren. In dem Verfahren von Figur 1 wird zusätzlich in den Schritten a) und e) Stickstoff als Inertgas eingespeist, um eine Bildung von brennbaren Gasgemischen zu verhindern. Dem ersten Festbettreaktor (a) werden Wasserstoffperoxid (1), Allylchlorid (2), Methanol (3) und Stickstoff (4) zugeführt. Die im ersten Festbettreaktor (a) erhaltene Reaktionsmischung wird in der Destillationskolonne (b) aufgetrennt in ein Kopfprodukt (7), das Allylchlorid und nicht umgesetzte Chlorpropane enthält (Mischung A), ein Sumpfprodukt (5), das Epichlorhydrin enthält (Mischung B), sowie einen Abgasstrom (6). Die Mischung A wird dann in (c) aufgeteilt in einen Strom (8), der in den ersten Festbettreaktor (a) zurückgeführt wird (Mischung A1) und einen Strom (9), der dem zweiten Festbettreaktor (d) zugeführt wird (Mischung A2) und dort mit weiterem Wasserstoffperoxid (10) umgesetzt wird. Die im zweiten Festbettreaktor (d) erhaltene Reaktionsmischung wird in der Destillationskolonne (e) aufgetrennt in ein Kopfprodukt (12), das die Chlorpropane enthält (Mischung C) und ein Sumpfprodukt (13), das Epichlorhydrin enthält (Mischung D) und in den ersten Festbettreaktor (a) zurückgeführt wird. Der Destillationskolonne (e) wird zusätzlich Stickstoff (11) zugeführt.

### Beispiele:

### Beispiel 1:

### Einfluss des Molverhältnisses von Allylchorid zu Wasserstoffperoxid auf die Selektivität der Umsetzung

Allylchlorid wurde mit Wasserstoffperoxid in Methanol als Lösungsmittel unter Verwendung eines Titansilicalit-Katalysators mit MFI-Struktur umgesetzt. Die Umsetzung erfolgte in zwei in Reihe geschalteten Rohrreaktoren, die über einen Kühlmantel gekühlt wurden. Der Katalysator wurde als Festbett in Form von Extrudaten eingesetzt. Der erste Reaktor enthielt 21,5 g Katalysator, der zweite Reaktor 20,7 g. Dem ersten Reaktor wurden Allylchlorid und eine Mischung aus einer wässrigen Wasserstoffperoxidlösung und Methanol kontinuierlich zugeführt. Die dosierten Mengenströme, die Zusammensetzung der Mischung und das molare Verhältnis von Allylchlorid zu Wasserstoffperoxid in den Edukten sind in Tabelle 1 wiedergegeben. Die reaktoren wurden in Aufstromfahrweise betrieben, wobei der Druck in den Reaktoren auf 7 bis 8 bar gehalten wurde und der erste Reaktor auf 36°C und der zweite Reaktor auf 38°C temperiert wurde. In der erhaltenen Reaktionsmischung wurde oder Gehalt an Wasserstoffperoxid durch Redoxtitration und die Gehalte an Allylchlorid und Epichlorhydrin durch Gaschromatographie bestimmt. Die aus diesen Gehalten berechneten Umsätze an Wasserstoffperoxid und Selektivitäten für Epichlorhydrin bezogen auf umgesetztes Allylchlorid sind in Tabelle 1 wiedergegeben. Tabelle 1 zeigt, dass mit zunehmendem molaren Überschuss an Allylchlorid die Selektivität für Epichlorhydrin zunimmt.

**Tabelle 1**

| Zusammensetzung der H₂O₂-MeOH-Mischung in Gew.-% | | | Dosierte Mengenströme, in 9/h | | Molverhältnis Allylchlorid/ H₂O₂ | H₂O₂-Umsatz in % | Selektivität für Epichlorhydrin in % |
|---|---|---|---|---|---|---|---|
| H₂O₂ | Wasser | MeOH | H₂O₂-MeOH-Mischung | Allylchlorid | | | |
| 10,5 | 7,9 | 81,1 | 73,1 | 28,7 | 1,7 | 84,5 | 88,3 |
| 8,4 | 6,3 | 85,3 | 72,8 | 29,0 | 2,1 | 85,1 | 88,8 |
| 8,4 | 6,3 | 85,3 | 73,2 | 43,3 | 3,1 | 85,8 | 91,0 |
| 10,5 | 7, 9 | 81,1 | 72,0 | 57,9 | 3,4 | 87,6 | 92,6 |
| 10,5 | 7, 9 | 81,1 | 64,4 | 58,3 | 3,8 | 88,5 | 92,9 |
| 8,4 | 6,5 | 85,0 | 73,0 | 58,1 | 4,2 | 90,3 | 93,2 |
| 8,0 | 5,9 | 86,2 | 72,7 | 71,8 | 5,5 | 88,0 | 94,4 |

### Beispiel 2:

### Verfahren gemäß Figur 1

Für das Verfahren von Figur 1 wurden die Umsetzungen in den Festbettreaktoren (a) und (d) in einem Rohrreaktor entsprechend Beispiel 1 für ein molares Verhältnis von Allylchlorid zu Wasserstoffperoxid von 4,0 in Reaktor (a) und 1,1 in Reaktor (b) experimentell nachgestellt. Der Reaktor enthielt dazu 42,6 g Katalysator und wurde auf 40°C temperiert. Die dosierten Mengenströme, die Zusammensetzung der Mischung aus Wasserstoffperoxid, Wasser und Methanol und das molare Verhältnis von Allylchlorid zu Wasserstoffperoxid in den Edukten sind in Tabelle 2 wiedergegeben, wobei die Werte so gewählt wurden, wie sie sich auf Grund der Rückführungen im Verfahren von Figur 1 ergeben. Mit den experimentell bestimmten Selektivitäten und einer Abschätzung für die Reaktivität von 1-Chlorpropen relativ zu Allylchlorid wurde dann für das Verfahren von Figur 1 mit dem Simulationsprogramm Aspen Plus der Firma aspentech die Zusammensetzung der Mengenströme 1 bis 13 berechnet. Die Resultate sind in Tabelle 3 wiedergegeben.

Bei dem Verfahren von Figur 1 werden zur Erstellung von einem Mol Epichlorhydrin 1,12 mol Allylchlorid benötigt. Bei einem Verfahren entsprechend dem Stand der Technik, bei dem die in Strom 9 enthaltene Menge an Allylchlorid aus dem Verfahren ausgeschleust wird, werden dagegen zur Herstellung von einem Mol Epichlorhydrin 1,35 mol Allylchlorid benötigt.

**Tabelle 2**

| Zusammensetzung der H₂O₂-MeOH-Mischung in Gew.-% | | | Dosierte Mengenströme in g/h | | Molverhältnis Allylchlorid/ H₂O₂ | Selektivität für Epichlorhydrin in % |
|---|---|---|---|---|---|---|
| H₂O₂ | Wasser | MeOH | H₂O₂-MeOH-Mischung | Allyl-chlorid | | |
| 7,7 | 7,9 | 84,4 | 80,2 | 58,2 | 4,0 | 92,9 |
| 6,7 | 6,2 | 85,1 | 106,6 | 23,7 | 1,1 | 87,9 |

**Tabelle 3**

| Komponente | Stoffstrom und Anteil der Komponente im Stoffstrom in g/h | | | | | | | | | | | | |
|---|---|---|---|---|---|---|---|---|---|---|---|---|---|
| | 1 | 2 | 3 | 4 | 5 | 6 | 7 | 8 | 9 | 10 | 11 | 12 | 12 |
| N₂ | 0,0 | 0,0 | 0,0 | 10,0 | 0,0 | 10,0 | 0,0 | 0,0 | 0,0 | 0,0 | 3,8 | 2,5 | 0,0 |
| O₂ | 0,0 | 0,0 | 0,0 | 0,0 | 0,0 | 1,7 | 0,0 | 0,0 | 0,0 | 0,0 | 0,0 | 0,4 | 0,0 |
| 2-Chlörpropan | 0,0 | 1,0 | 0,0 | 0,0 | 0,0 | 0,0 | 9,9 | 8,9 | 1, 0 | 0,0 | 0,0 | 1,0 | 0,0 |
| 1-Chlorpropen | 0,0 | 0,7 | 0,0 | 0,0 | 6,0 | 0,0 | 6,4 | 5,9 | 0,5 | 0,0 | 0,0 | 0,5 | 0,0 |
| Allylchlorid | 0,0 | 464,9 | 0,0 | 0,0 | 0,0 | 0,0 | 1190,1 | 1101,0 | 89,1 | 0,0 | 0,0 | 8,9 | 0,0 |
| 1-Chlörpropan | 0,0 | 1,1 | 0,0 | 0,0 | 0,0 | 0,0 | 14,7 | 13,6 | 1,1 | 0,0 | 0,0 | 1,1 | 0,0 |
| MeOH | 0,0 | 0,0 | 17,2 | 0,0 | 0,4 | 0,0 | 771,7 | 713,9 | 57,7 | 0,0 | 0,0 | 0,9 | 52,8 |
| Wasser | 75,0 | 0,0 | 0,0 | 0,0 | 201,0 | 0,0 | 4,9 | 4,5 | 0, 4 | 15,5 | 0,0 | 0,0 | 35,0 |
| Epichlorhydrin | 0,0 | 0,0 | 0,0 | 0,0 | 500,4 | 0,0 | 17, 0 | 15,7 | 1, 3 | 0,0 | 0,0 | 0,0 | 84,3 |
| H₂O2 | 175,0 | 0,0 | 0,0 | 0,0 | 0,0 | 0,0 | 0,0 | 0,0 | 0,0 | 36,2 | 0,0 | 0,0 | 0,0 |
| 3-Chlor-1-methoxy-2-propanol | 0,0 | 0,0 | 0,6 | 0,0 | 64,5 | 0,0 | 0,0 | 0,0 | 0,0 | 0,0 | 0,0 | 0,0 | 17,7 |

## Patentansprüche

1. Verfahren zur Herstellung von Epichlorohydrin, wobei
a) in einer ersten Reaktionsstufe Allylchlorid und Wasserstoffperoxid in Gegenwart eines titanhaltigen Zeolithkatalysators in einem molaren Verhältnis von Allylchlorid zu Wasserstoffperoxid von mindestens 1,5:1 umgesetzt werden und wobei das eingesetzte Allylchlorid 1-Chlorpropan und/oder 2-Chlorpropan enthält,
b) die in der ersten Reaktionsstufe gebildete Reaktionsmischung in einer Destillation aufgetrennt wird in eine Mischung (A), die nicht umgesetztes Allylchlorid, sowie 1-Chlorpropan und/oder 2-Chlorpropan enthält und eine Mischung (B), die Epichlorhydrin enthält,
c) die Mischung (A) aufgeteilt wird in eine Mischung (A1), die in die erste Reaktionsstufe zurückgeführt wird und eine Mischung (A2),
d) die Mischung (A2) in einer zweiten Reaktionsstufe mit Wasserstoffperoxid in Gegenwart eines titanhaltigen Zeolithkatalysators in einem molaren Verhältnis von Allylchlorid zu Wasserstoffperoxid im Bereich von 0,5:1 bis 1,25:1 umgesetzt wird,
e) die in der zweiten Reaktionsstufe gebildete Reaktionsmischung in einer Destillation aufgetrennt wird in eine Mischung (C), die 1-Chlorpropan und/oder 2-Chlorpropan enthält und eine Mischung (D), die Epichlorhydrin enthält und
f) die Mischung (C) aus dem Verfahren entnommen wird.

2. Verfahren nach Anspruch 1,
**dadurch gekennzeichnet,**
**dass** die Umsetzung von Allylchlorid und Wasserstoffperoxid in den Schritten a) und d) in Gegenwart eines Lösungsmittels erfolgt.

3. Verfahren nach Anspruch 2,
**dadurch gekennzeichnet,**
**dass** das Lösungsmittel Methanol ist.

4. Verfahren nach Anspruch 3,
**dadurch gekennzeichnet,**
**dass** Wasserstoffperoxid in Form einer Lösung von Wasserstoffperoxid in Methanol eingesetzt wird.

5. Verfahrens nach einem der vorangehenden Ansprüche,
**dadurch gekennzeichnet,**
**dass** in Schritt a) das molare Verhältnis von Allylchlorid zu Wasserstoffperoxid im Bereich von 1,5:1 bis 5:1 liegt.

6. Verfahren nach einem der vorangehenden Ansprüche,
**dadurch gekennzeichnet,**
**dass** die Aufteilung der Mischung (A) in Schritt c) durch eine Destillation erfolgt, bei der die in Mischung (A) enthaltenen Chlorpropane in der Mischung (A2) angereichert werden.

7. Verfahren nach einem der vorangehenden Ansprüche,
**dadurch gekennzeichnet,**
**dass** die Mischung (D) in die erste Reaktionsstufe zurückgeführt wird.

8. Verfahren nach Anspruch 7,
**dadurch gekennzeichnet,**
**dass** die erste Reaktionsstufe in zwei in Reihe geschalteten Reaktoren durchgeführt wird und die Mischung (D) in den zweiten Reaktor zurückgeführt wird.

9. Verfahren nach einem der vorangehenden Ansprüche,
**dadurch gekennzeichnet,**
**dass** die Schritte d) und e) gleichzeitig in einer Reaktivdestillation erfolgen.

## Claims

1. Process for the preparation of epichlorohydrin where
a) in a first reaction stage, allyl chloride and hydrogen peroxide are reacted in the presence of a titanium-containing zeolite catalyst in a molar ratio of allyl chloride to hydrogen peroxide of at least 1.5:1 and where the allyl chloride used is 1-chloropropane and/or 2-chloropropane,
b) the reaction mixture formed in the first reaction stage is separated in a distillation into a mixture (A) which comprises unreacted allyl chloride, and also 1-chloropropane and/or 2-chloropropane, and a mixture (B) which comprises epichlorohydrin,
c) the mixture (A) is divided into a mixture (A1), which is returned to the first reaction stage, and a mixture (A2),
d) the mixture (A2) is reacted in a second reaction stage with hydrogen peroxide in the presence of a titanium-containing zeolite catalyst in a molar ratio of allyl chloride to hydrogen peroxide in the range from 0.5:1 to 1.25:1,
e) the reaction mixture formed in the second reaction stage is separated in a distillation into a mixture (C), which comprises 1-chloropropane and/or 2-chloropropane, and a mixture (D), which comprises epichlorohydrin and
f) the mixture (C) is removed from the process.

2. Process according to Claim 1,
**characterized in that**
the reaction of allyl chloride and hydrogen peroxide in steps a) and d) takes place in the presence of a solvent.

3. Process according to Claim 2,
**characterized in that**
the solvent is methanol.

4. Process according to Claim 3,
**characterized in that**
hydrogen peroxide is used in the form of a solution of hydrogen peroxide in methanol.

5. Process according to one of the preceding claims,
**characterized in that**
in step a) the molar ratio of allyl chloride to hydrogen peroxide is in the range from 1.5:1 to 5:1.

6. Process according to one of the preceding claims,
**characterized in that**
the division of the mixture (A) in step c) takes place by a distillation in which the chloropropanes present in mixture (A) are enriched in the mixture (A2).

7. Process according to one of the preceding claims,
**characterized in that**
the mixture (D) is returned to the first reaction stage.

8. Process according to Claim 7,
**characterized in that**
the first reaction stage is carried out in two reactors connected in series and the mixture (D) is returned to the second reactor.

9. Process according to one of the preceding claims,
**characterized in that**
steps d) and e) take place simultaneously in a reactive distillation.

## Revendications

1. Procédé pour la production d'épichlorhydrine, dans lequel
a) dans une première étape de réaction on fait réagir du chlorure d'allyle et du peroxyde d'hydrogène en présence d'un catalyseur zéolithique contenant du titane, en un rapport molaire du chlorure d'allyle au peroxyde d'hydrogène d'au moins 1,5:1 et dans lequel le chlorure d'allyle utilisé contient du 1-chloropropane et/ou du 2-chloropropane,
b) dans une distillation on fractionne le mélange réactionnel, formé dans la première étape de réaction, en un mélange (A) qui contient du chlorure d'allyle n'ayant pas réagi, ainsi que du 1-chloropropane et/ou du 2-chloropropane, et un mélange (B) qui contient de l'épichlorhydrine,
c) on divise le mélange (A) en un mélange (A1), qui est renvoyé dans la première étape de réaction, et un mélange (A2),
d) dans une deuxième étape de réaction on fait réagir le mélange (A2) avec du peroxyde d'hydrogène en présence d'un catalyseur zéolithique contenant du titane, en un rapport molaire du chlorure d'allyle au peroxyde d'hydrogène dans la plage de 0,5:1 à 1, 25: 1,
e) dans une distillation on fractionne le mélange réactionnel, formé dans la deuxième étape de réaction, en un mélange (C), qui contient du 1-chloropropane et/ou du 2-chloropropane, et un mélange (D) qui contient de l'épichlorhydrine et
f) on prélève le mélange (C) du procédé.

2. Procédé selon la revendication 1, **caractérisé en ce que** la réaction du chlorure d'allyle et du peroxyde d'hydrogène dans les étapes a) et d) s'effectue en présence d'un solvant.

3. Procédé selon la revendication 2, **caractérisée en ce que** le solvant est le méthanol.

4. Procédé selon la revendication 3, **caractérisé en ce que** le peroxyde d'hydrogène est utilisé sous forme d'une solution de peroxyde d'hydrogène dans du méthanol.

5. Procédé selon l'une quelconque des revendications précédentes, **caractérisé en ce que** dans l'étape a) le rapport molaire du chlorure d'allyle au peroxyde d'hydrogène se situe dans la plage de 1,5:1 à 5:1.

6. Procédé selon l'une quelconque des revendications précédentes, **caractérisé en ce que** la division du mélange (A) dans l'étape c) s'effectue par une distillation, dans laquelle les chloropropanes contenus dans le mélange (A) sont concentrés dans le mélange (A2).

7. Procédé selon l'une quelconque des revendications précédentes, **caractérisé en ce que** le mélange (D) est renvoyé dans la première étape de réaction.

8. Procédé selon la revendication 7, **caractérisé en ce que** la première étape de réaction est effectuée dans deux réacteurs raccordés en série et le mélange (D) est renvoyé dans le second réacteur.

9. Procédé selon l'une quelconque des revendications précédentes, **caractérisé en ce que** les étapes d) et e) s'effectuent simultanément dans une distillation réactive.
